Europäisches Patentamt

European Patent Office

Office européen des brevets

(19)

(11) Publication number:
**0 335 977**
**A1**

(12)

## EUROPEAN PATENT APPLICATION
## published in accordance with Art.
## 158(3) EPC

(21) Application number: 88907785.5

(22) Date of filing: 09.09.88

(86) International application number:
PCT/JP88/00906

(87) International publication number:
WO 89/02245 (23.03.89 89/07)

(51) Int. Cl.⁴: **A61B 5/04**

(30) Priority: 19.09.87 JP 235705/87
04.08.88 JP 195341/88

(43) Date of publication of application:
11.10.89 Bulletin 89/41

(84) Designated Contracting States:
AT BE CH DE FR GB IT LI LU NL SE

(71) Applicant: KAWABE, Jiro
91, Azahamazaki, Oazamugiura, Mugicho
Kaifu-gun, Tokushima 775(JP)

Applicant: AKAMATSU, Norio
Room 605, No. 5, Miyake Bldg., 9-3,
Sumiyoshi, 4-chome
Tokushima-shi, Tokushima 770(JP)

Applicant: TOYOSU, Yasuhiro
15-5, Minamikomatsushimacho
Komatsushima-shi
Tokushima 773(JP)

(72) Inventor: AKAMATSU, Norio Room 605 No. 5
Miyake Bldg. 9-3, Sumiyoshi 4-chome
Tokushima-shi Tokushima 770(JP)
Inventor: TOYOSU, Yasuhiro
15-5, Minamikomatsushimacho
Komatsushima-shi Tokushima 773(JP)

(74) Representative: Lehn, Werner, Dipl.-Ing.
Hoffmann, Eitle & Partner Patentanwälte
Arabellastrasse 4
D-8000 München 81(DE)

(54) METHOD OF MEASURING BIOPOTENTIAL.

(57) A method of measuring biopotentials wherein an electrode is brought into contact with or is brought close to a living body in order to detect an electrical signal generated in the living body based on a potential induced on the electrode. To inspect the condition where the electrode is contacted, a refer- ence signal is given to the living body. The electrode detects the reference signal of a human body. The reference signal measured from the human body by the electrode represents the condition where the electrode is contacted. The electrode detects the reference signal and an electric signal generated in

the living body. The signal level of the living body decreases with the decrease in the reference signal level induced on the electrode. Therefore, the signal level of the living body can be corrected with the reference signal level induced on the electrode.

FIG. 1

FIG. 2

# METHOD FOR POTENTIAL MEASUREMENT ON A LIVING BODY

## FIELD OF THE INVENTION

This invention relates primarily to a method for measuring electrical potential appearing at the surface of a living body for use in electrocardiographs and electroencephalographs.

## BACKGROUND OF THE INVENTION

In electrocardiographs and electroencephalographs, electrical potential at the surface of a living body is measured by electrodes in contact with that body. To obtain accurate measurements, it is important that contact resistance between the body surface and contacting electrodes is sufficiently low. Nevertheless, there is large contact resistance variation with electrode location on the body surface. There is also large variation in contact resistance from one subject to another. The measurement error produced by contact resistance variation is reduced by making the input impedance of the first stage amplifier connected to an electrode, sufficiently high compared with the contact resistance. However, a high input impedance first stage amplifier has a high susceptibility to noise. It is also difficult to retain high input impedance over long periods, and impedance decay with time gives rise to measurement error.

In actual body potential measurement, it is difficult to sufficiently lower the electrode to body contact resistance compared to the input impedance of the first stage amplifier. This is consequently a source of error in body potential measurement. Particularly in electrocardiograph instruments, which simultaneously measure body potential at numerous points and display a potential map of the body surface, measurement error at individual points greatly influences the map displayed. It is also extremely difficult to make accurate potential measurements simultaneously at a multiplicity of points on the body surface.

Non contacting insulated electrodes have also been developed. These electrodes lie flat on the body surface, but a dielectric prevents direct electrical contact with the body. The capacitor created by the body surface, the dielectric, and the planar electrode acts to induce body potential at the electrode.

The low frequency limit for insulated electrodes is determined by the electrode capacitance and the input impedance of the head amplifier (buffer amplifier at, or close to the electrode). Namely, the larger the electrode capacitance and head amplifier input impedance, the lower the frequency of signals that can be detected from the body. However, as for contacting electrodes, body potential varies with the state of attachment to the body surface for electrodes of this configuration as well. Whenever the dielectric is not firmly in contact with the body surface, the body potential detected by the electrode is reduced.

Consequently, with non contacting electrodes as well, body potential cannot be consistently and accurately measured.

The present invention was developed to eliminate the deficiencies mentioned above. It is thus the primary object of the present invention to provide a method for accurately measuring electrical potential on a living body by applying a calibration signal which is measured at electrodes to determine the accuracy of the body potential detected at those electrodes.

## DISCLOSURE OF THE INVENTION

This invention provides a method for potential measurement on a living body, wherein voltage signals produced in the body are detected by electrodes which contact or lie very close to the body surface and induce the body's electrical potential. In this method for potential measurement, a calibration signal is applied to the living body, is detected at the electrodes, and is used to examine the electrodes' body potential signal detection capability.

The method for potential measurement on a living body of this invention is particularly suited for measuring induced potential at a multiplicity of points on the human body. Simultaneously maintaining all of the electrodes at a multiplicity of points on the human body in a state of contact favorable to body potential measurement is difficult. It is also difficult to determine the state of contact of an electrode just from the body potential signal picked up at that electrode. In this invention, a calibration signal is applied to the human body. The applied calibration signal is detected at each electrode, and the calibration signal picked up by an electrode indicates the electrical state of contact of that electrode. Hence, the state of electrical contact to the human body of a multiplicity of electrodes can be investigated by detection of a calibration signal applied to the body. The method of this invention is, therefore, effective with the use of numerous electrodes for the measurement of signals from a living body.

## BRIEF DESCRIPTION OF THE DRAWINGS

FIG. 1 is a block diagram showing an embodiment of a system employing the method for potential measurement on a living body of the present invention;

FIG. 2 is block diagram showing an amplifier depicted in FIG. 1;

FIG. 3 is graphs showing the electrocardiograph waveform picked up at an electrode, the calibration signal picked up at an electrode, the compensated electrocardiograph signal, and the output waveform of the comparator;

FIG. 4 is a block diagram showing another embodiment of a system employing the method of the present invention;

FIG. 5 is a block diagram showing an amplifier depicted in FIG. 4;

FIG. 6 through FIG. 9 are graphs showing the electrocardiograph and calibration waveforms picked up at an electrode;

FIG. 10 is a block diagram showing an alternative circuit to that shown in FIG. 5;

FIG. 11 and FIG. 12 are block diagrams showing other embodiments of the application of a calibration signal to.the human body.

## DESCRIPTION OF THE PREFERRED EMBODIMENT

The preferred embodiment of the present invention is described based on illustrations as follows.

The method for potential measurement on a living body of the present invention is primarily employed in the measurement of electrocardiograph and electroencephalograph signals appearing at the surface of the human body. However, the method of the present invention can be used in the measurement of any voltage signals at the surface of the living body. For example, it can be used for measurement of signals such as electromyograph signals. The method of the present invention is particularly suited for use in systems which simultaneously measure body potential at a multiplicity of points, and either display the relative potential of neighboring electrodes, or display equipotential maps con structed from all the measured signals.

A preferred embodiment of the method of the present invention as applied to the measurement of electrocardiograph signals follows. However, this method can be used in essentially the same manner in the measurement of electroencephalograph or electromyograph signals as well.

The method for potential measurement on a living body of this invention is particularly well suited for systems which detect signals at a multiplicity of points on a living body. For example, it is very well suited for body surface electrocardiograph machines which detect electrocardiograph signals at a multiplicity of points on the body surface.

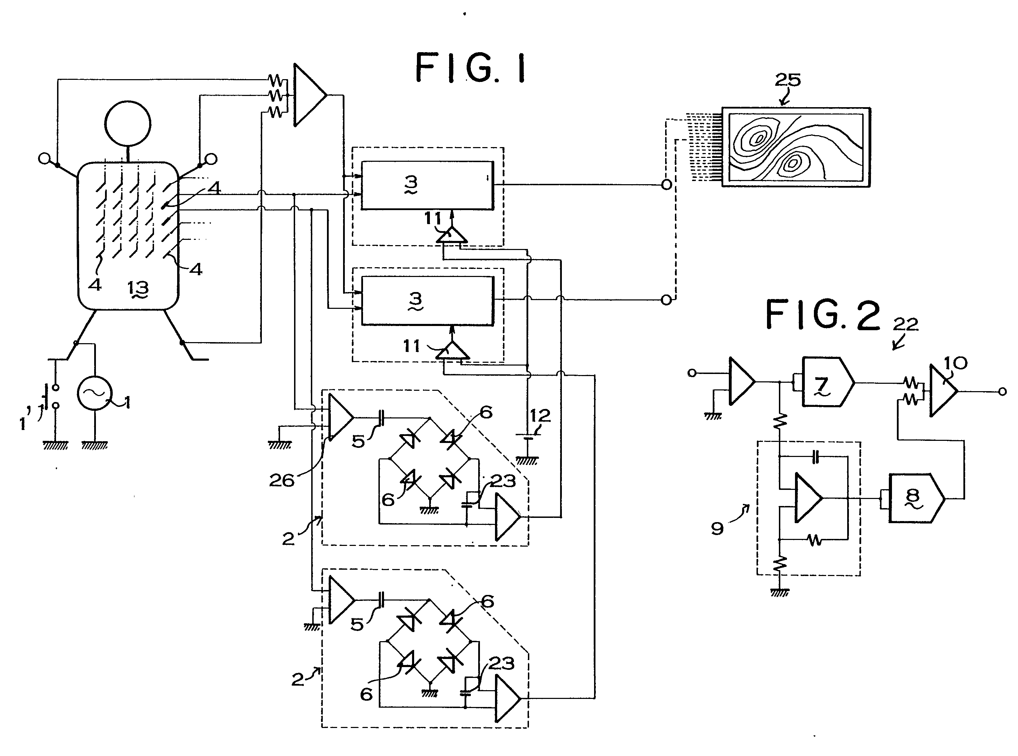

A body surface electrocardiograph system typically measures electrocardiograph potential at 40 to 1000 points on the body surface. The electrocardiograph potential is measured at each electrode. Consequently, 40 to 1000 electrodes are typically put in contact with the body surface. The electrocardiograph signal measured at each electrode is converted from analog to digital and input to a computer. The computer processes the input signals and, for example, computes a potential distribution map over the body surface as shown in FIG. 1. The potential distribution map is output to a display, printer, or plotter.

If any electrode is not sufficiently in contact with the body surface, an erroneous potential is picked up at that electrode, and distortion arises in the body surface potential distribution map. Furthermore, it is impossible by examining the body surface potential distribtuion map, to determine if the map distortion is caused by measurement error or by actual body potential. A method of determining the state of electrical contact of an electrode follows.

As shown in the method for electrocardiograph measurement depicted in Fig. 1, a calibration signal is applied between the human subject and ground. The calibration signal and the electrocardiograph signal are detected at an electrode 4. The calibration signal is amplified by a calibration signal amplifier 2, and the electrocardiograph potential is amplified by an electrocardiograph amplifier 3. The input side of the calibration signal amplifier 2 is provided with a differential amplifier 26 which measures the potential between the human body and ground. The electrocardiograph potential with respect to a reference point on the body is input to the electrocardiograph amplifier 3.

The calibration signal detected by an electrode 4 is input to the calibration signal amplifier 2, where it is amplified to determine the state of electrical contact of that electrode. An alternating current signal such as a sine wave or square wave is used as the calibration signal for eliminating the effects of measurement error caused by contact potential drop between the body surface 13 and an electrode 4.

An alternating current signal with very little amplitude variation is used as the calibration signal applied to the human body. This is necessary because the calibration amplifier 2 determines the state of electrical contact of an electrode 4 by detecting the amplitude of the input signal. However, for the case where all the electrodes 4 simultaneously pick up the calibration signal, it is also possible to apply signals with some amplitude variation to the body. This is because relative values of the calibration signal at all electrodes can be used to determine the state of electrical contact of each electrode. For example, if the calibration signal amplitude goes lower than a standard value, and if all electrodes are in good contact, all electrodes will measure the calibration signal amplitude lower than the standard value, but they will all measure the same amplitude signal.

The frequency of the calibration signal is selected to lie within the bandwidth for amplification by a low frequency calibration signal amplifier 2. For the case where a low frequency operational amplifier is used for the calibration signal amplifier 2, signals having a maximum frequency on the order of from 10 KHz to 100 KHz can be amplified. Accordingly, the frequency of the calibration signal generation (oscillator) 1 is selected below several KHz to several 100 KHz. However, for the case where a device capable of high frequency amplification is used for the calibration signal amplifier 2, it is possible to set the calibration signal frequency higher, for example, between 500 KHz and several MHz. Further to avoid signals which have too low a frequency and too long a period, the calibration signal is normally chosen to be above 1 Hz and preferably above 10 Hz.

Ideally, the frequency of the calibration signal is set equal to the frequency of the system power supply, for example, from 50 to 60 Hz. Choice of this frequency is effective in reducing noise susceptibility. This is because the period of the calibration signal and that of most of the induced noise is identical when the calibration signal frequency is the same as that of the power supply. That is, when the period of noise picked up by an electrode or a head amplifier (buffer amplifier) is equal to that of the calibration signal, the noise is masked by the calibration signal measured at the electrode.

The peak to peak voltage of the calibration signal applied to the human body by the signal generator (oscillator) 1 is such that the signal to noise ratio of the detected calibration signal is sufficiently high. Normally this voltage is selected to be 2 mV or greater, preferably 5 mV or greater, and ideally 10 mV or greater.

If the amplitude of the calibration signal is too large, clipping will occur in the calibration signal amplifier 2. For the case where the calibration signal is applied directly to the human body, as shown in FIG. 1, the peak to peak output voltage of the calibration signal generator (oscillator) 1 is adjusted at or below 20 V, and preferably at or below 10 V.

The input impedance of the calibration signal amplifier 2 is selected to be sufficiently large compared with the contact resistance of the electrode with the body surface. For example, the input impedance is normally selected greater than or equal to 500 KΩ, preferably greater than or equal to 1 MΩ, and ideally greater than or equal to 5 MΩ. The calibration signal amplifier 2 input impedance is determined by the type of electrode used, or namely, the contact resistance between the electrode and the body surface. For electrodes with low contact resistance values, input impedances below 500 KΩ can be used. Calibration signal amplifiers with low input impedance are able to measure the calibration signal with low noise susceptibility.

If the input impedances of the calibration signal amplifier 2 and the electrocardiograph amplifier 3 are made equal, these amplifiers can measure the calibration signal and the electrocardiograph potential detected at an electrode 4 under the same conditions.

The output impedance of the calibration signal amplifier 2 is selected to be sufficiently low, preferably less than or equal to 1 KΩ.

The calibration signal amplifier 2 measures the amplitude of the calibration signal detected at an electrode 4 by eliminating any direct current component with a coupling capacitor 5 connected to the output side of the differential amplifier 26, and then rectifying this signal with a diode bridge 6. The measured calibration signal rectified by the diode bridge 6 is filtered by charging the smoothing capacitor 23. The output from the smoothing capacitor 23 is proportional to the peak value of the calibration signal measured by the electrode 4.

In the case where the calibration signal is a sine wave, it is possible to continuously monitor the amplitude of the calibration signal measured at an electrode 4. A calibration signal amplifier 22 capable of continuously monitoring the amplitude of the calibration signal is shown in FIG. 2. This calibration signal amplifier 22 makes use of the trigonometric identity $A^2\sin^2\theta + A^2\cos^2\theta = A^2$. Specifically, the calibration signal sine wave ($A\sin\theta$) measured at an electrode 4 is squared by a squaring circuit 7 to obtain the signal $A^2\sin^2\theta$. The sine wave is also input to an integrator 9 to obtain a cosine wave ($A\cos\theta$) which is 90° out of phase with the input signal. The cosine wave is squared by the squaring circuit 8 to obtain the signal $A^2\cos^2\theta$. The outputs of the two squaring circuits 7 and 8 are then added in the adder 10 to produce the signal $A^2\sin^2\theta + A^2\cos^2\theta = A^2$. This output signal contains no alternating current components, and corresponds to the amplitude (A). Since this circuit outputs a direct current level corresponding to the amplitude of the calibration signal detected an an electrode, the state of electrical contact of that electrode can be continuously monitored.

As described above, in the method for potential measurement on a living body of this invention calibration signal is applied to the body, and both the calibration signal and the body potential signal are detected at the electrodes. For this reason, when an electrode is in poor electrical contact with the body, and body potential cannot be accurately measured, the measured amplitude of the calibration signal is also reduced. Therefore, whether or not an electrode can accurately measure body potential, can be simply discerned by the calibration signal measured at that electrode.

Suppose that an electrode is not completely in contact with the body, and the measured body potential is only half of its true value. In this case, the amplitude of the calibration sig nal detected by that electrode will also be reduced by half. In other words, the fact that an electrode's contact is poor, and only half the body potential signal is measured, can be determined from the calibration signal detected at that electrode.

Hence, the state of electrical contact between an electrode and the body can be accurately determined by detection of the calibration signal at that electrode.

In the method for potential measurement on a living body of this invention, a calibration signal of uniform amplitude is applied to the human body, and the state of electrical contact of the electrodes is determined by the amplitude of the calibration signal measured at those electrodes. The amplitude of the calibration signal detected at an electrode, in other words, what kind of electrical contact is made between the electrode and the body, can be used to control the gain of the electrocardiograph amplifier. If this is done, correct body potential values can be obtained from electrodes which are not in good contact with the body. This is realized, as shown in FIG. 1, by an electrocardiograph amplifier 3 that has its gain controlled by the output voltage of the calibration signal amplifier 2.

When an electrode is in good electrical contact with the body, in other words, when an electrode measures the prescribed calibration signal amplitude, the electrocardiograph amplifier 3 amplifies the detected electrocardiograph potential with a gain which is equal to a pre-set value such as one or two thousand. If an electrode 4 is in poor contact with the body, and the amplitude of the detected calibration signal is reduced, the electrocardiograph potential measured by the electrode is also reduced. When an electrode's contact to the body becomes poor, and the amplitude of the calibration signal detected at that electrode 4 decreases below the prescribed amplitude, the electrocardiograph amplifier 3 is controlled such that its gain is increased. The gain of the electrocardiograph amplifier 3 is controlled by the output of the calibration signal amplifier 2. When calibration signal amplifier output decreases below the prescribed value, electrocardiograph amplifier 3 gain is increased to a value greater than one or two thousand. Consequently, the electrocardiograph amplifier 3 is provided with a voltage reference 12 to determine whether or not the calibration signal amplitude is equal to the prescribed value. The voltage reference 12 is compared to the calibration signal amplifier output in a comparator 11. The comparator 11 output regulates the gain of the electrocardiograph amplifier 3. The reference voltage 12 is adjusted to be equal to the calibration signal amplifier 2 output when the electrode 4 is in proper contact with the body surface. In other words, the output of the comparator 11 is zero when the electrode 4 properly measures body potential, and increases with degradation of electrode to body contact and reduction in calibration signal amplitude. The larger the comparator 11 output, the greater the electrocardiograph amplifier 3 gain.

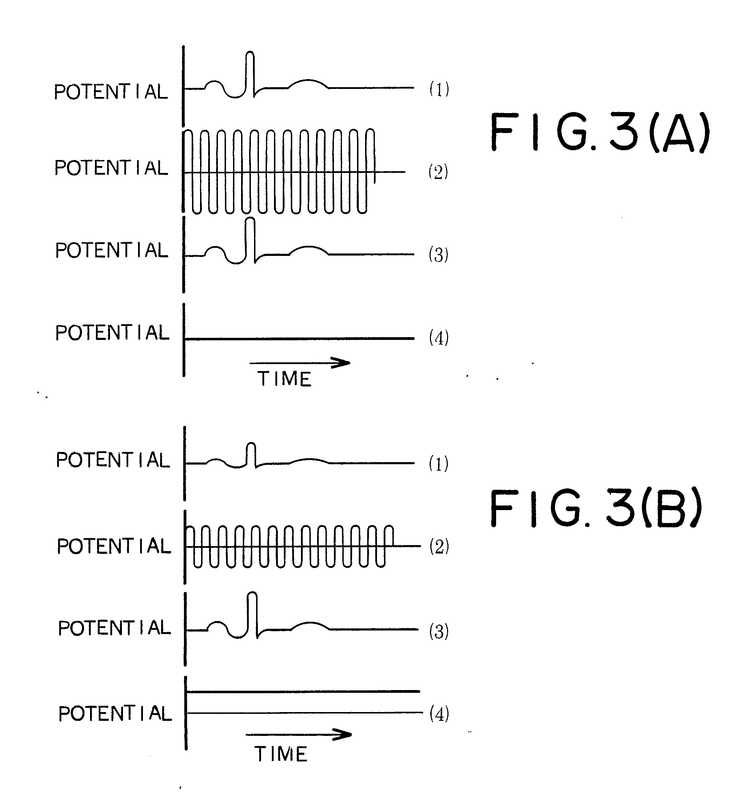

This situation is explained by FIG. 3. FIG. 3(A) depicts correct measurement of body potential by an electrode, while FIG. 3(B) depicts a low measured body potential by an electrode in poor contact with the body. In these graphs, (1) is the electrocardiograph potential detected by an electrode, (2) is the calibration signal detected by that electrode, (3) is the output of the electrocardiograph amplifier 3 which is compensated by the calibration signal amplitude, and (4) is the comparator 11 output.

As shown in FIG. 3(A)(4), when an electrode detects the correct body potential, the comparator output is zero, and the electrocardiograph amplifier amplifies the body potential signal with the pre-set gain.

As shown in FIG. 3(B)(2), when the calibration signal amplitude decreases, the comparator output shown in (4) rises, and this output adjusts the gain of the electrocardiograph amplifier higher to compensate for the lower measured electrocardiograph potential.

The electrocardiograph amplifier 3 (including the comparator 11) is a automatic gain control amplifier with its gain controlled to be inversely proportional to the amplitude of the calibration signal detected by the calibration signal amplifier 2. For example, when the calibration signal amplifier 2 output is half the prescribed value, the gain of the electrocardiograph amplifier 3 is made twice the pre-set gain, and when the calibration signal amplitude is one third the prescribed value, the gain of the electrocardiograph amplifier 3 is made three times the pre-set gain. Since the calibration signal and the electrocardiograph potential are measured together at the same electrode, if that electrode's contact becomes poor and the amplitude of the calibration signal measured drops by one half, the measured value of the electrocardiograph potential also drops by one half. Hence, when the amplitude of the calibration signal is measured at the electrode to be one half the prescribed value, the correct body potential can be calculated by amplifying the electrocardiograph potential with twice the pre-set gain.

In the measurement method depicted in FIG. 1, the electrocardiograph potential and the calibration signal can be measured simultaneously. However, for continuous measurement of the calibration signal together with the electrocardiograph potential, the calibration signal should be selected such that it does not affect the electrocardiograph signal. For example, the amplitude of the calibration signal applied to the human body can be set very low, and a low noise amplifier can be used as the calibration signal amplifier, or the frequency of the calibration signal can be set sufficiently high compared to the frequency of the electrocardiograph signal, and a filter can be provided in the electrocardiograph amplifier to eliminate the calibration signal. The circuit shown in FIG. 1 can correct the electrocardiograph signal continuously, or very nearly continuously, while simultaneously detecting the amplitude of the calibration signal.

In actual electrocardiograph measurement, large variation in the state of electrode contact over short time intervals is seldom observed. For this reason it is also possible to periiodically sample the state of electrode contact at fixed periods of time, after each sampling measure the electrocardiograph potential, and correct the electrocardiograph potential based on the previously sampled calibration signal. Namely, it is possible to time division multiplex the calibration signal and electrocardiograph measurements.

In the case where electrocardiograph potential and calibration signal measurements are time division multiplexed using the measurement circuit shown in FIG. 1, there is no need to apply the calibration signal to the human body while electrocardiograph potential measurements are being taken. Accordingly, as shown in FIG. 1, a shorting switch 1' is connected to the output side of the calibration signal generator 1. When the electrocardiograph potential is being measured, the shorting switch is closed, or signal generation by the calibration signal generator 1 is stopped.

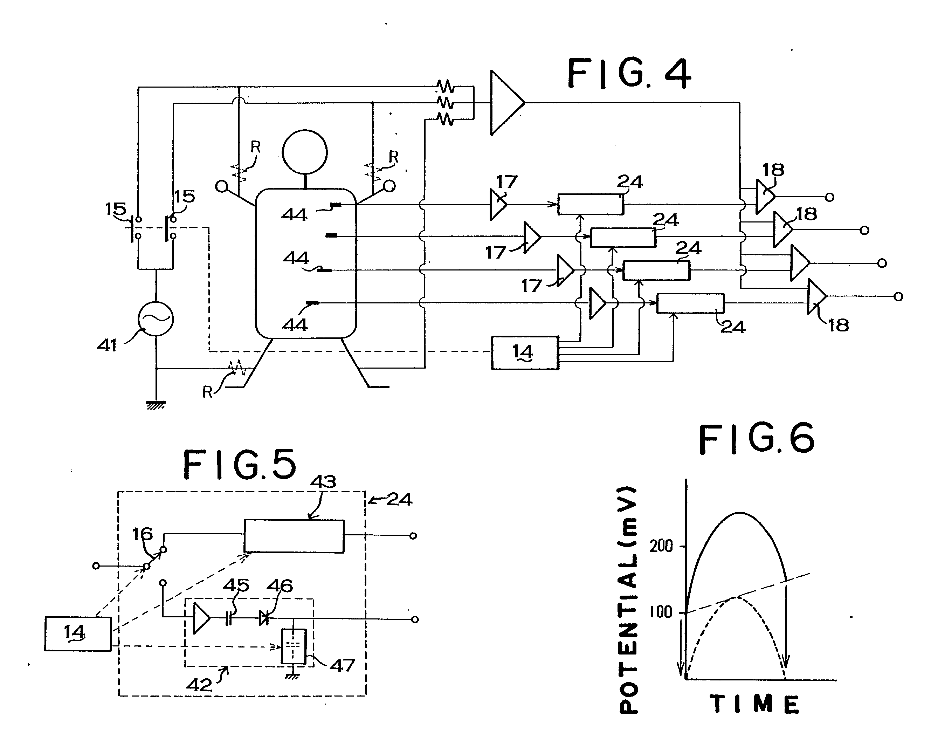

In the present invention, the method of applying a calibration signal to the human body is not limited to the configuration shown in FIG. 1. An alternate embodiment of the application of a calibration signal to the human body is shown in FIG. 4. This system is provided with buffer amplifiers 17 connected to the electrodes 44. Both the calibration signal and the electrocardiograph signal measured at an electrode 44 are input to, and amplified by the buffer amplifier 17. Accordingly, this system features the capability to measure both the calibration signal and the electrocardiograph signal under equal conditions.

In the circuit shown in FIG. 4, the calibration signal and the electrocardiograph signal are measured by time division multiplexing. Further, calibration signal measurements in this circuit are made by causing a weak current, corresponding to the calibration signal, to flow through the human body.

In the calibration signal measurement method shown in the circuit of FIG. 4, the calibration signal generator 41 output is applied between the right leg and both arms of the human body. Specifically, the calibration signal is applied to the body by passing current between the right leg and both arms. Both the electrocardiograph potential and the calibration signal are detected by the electrodes 44, and amplified by dual purpose amplifiers 24.

Each dual purpose amplifier 24 amplifies either the calibration signal or the electrocardiograph potential depending on time division multiplex switching. Specifically, after the calibration signal is detected indicating the state of electrical contact of an electrode 44, the electrocardiograph potential is measured and corrected using the measured amplitude of the calibration signal. The dual purpose amplifier 24 is switched by a control circuit 14 to amplify either the calibration signal or the electrocardiograph potential. The control circuit 14 applies the calibration signal to the human body only when the dual purpose amplifier 24 is switched to amplify the calibration signal. For this purpose, switching devices 15 are connected between the output of the calibration signal generator 41 and both of the subject's arms. The switching devices 15 are switched by the control circuit 14, and are turned on only during calibration signal measurement.

Details of the dual purpose amplifier 24 of FIG. 4, are shown in FIG. 5. Each dual purpose amplifier 24 is provided with an electrocardiograph amplifier 43, a calibration signal amplifier 42, and an input select switch 16. The input select switch 16 is switched by the control circuit 14. When the input select switch 16 is in the solid line position shown in FIG. 5, the signal detected by an electrode 44 is input to the electrocardiograph amplifier, and when the input select switch 16 is in the other position, the signal detected by the electrode 44 is input to the calibration signal amplifier 41.

The calibration signal amplifier 42 amplifies the input calibration signal, and eliminates the direct current (DC) component with the capacitor 45 at the output. The alternating current calibration signal with DC levels eliminated, is converted to a DC level proportional to the amplitude of the calibration signal by the rectifying diode 46. Since the diode 46 DC output voltage 46 is proportional to the amplitude of the calibration signal detected at an electrode, the state of electrical contact of the electrode can be verified with that output signal.

The diode 46 output voltage, which is the output of the calibration signal amplifier, can be observed on an oscilloscope, video display, or printed hardcopy. This allows the electrode's state of electrical contact to be determined. In the case where the calibration signal amplifier 42 output is observed on a video display or confirmed with a hardcopy, the input or output side of the capacitor 45 may be observed instead.

When electrode contact is confirmed by video display or hardcopy, the state of contact of all electrodes is checked by applying a calibration signal to the human body, then after good contact with the body has been confirmed, application of the calibration signal is ceased, and the electrocardiograph potential is measured.

Electrode contact can also be confirmed by video display or printout using the circuit of FIG. 1. In this case, the output of the differential amplifier 26 is observed.

Systems in which the electrocardiograph potential is measured after confirming electrode contact by video display or printer, can only obtain accurate data when all electrodes are in favorable contact with the body. Systems in which the electrocardiograph amplifier gain is controlled by the calibration signal, can obtain accurate data even when some electrodes are in somewhat less than favorable cotnact with the body.

In the same manner as the system of FIG. 1, the gain of the electrocardiograph amplifier 43 of the circuit shown in FIG. 4 and FIG. 5 can be controlled according to the state of contact of the electrode. When this is done, an automatic gain control circuit is used in the electrocardiograph amplifier 43, just as it was used in the electrocardiograph amplifier 3 of FIG. 1. The gain of the electrocardiograph amplifier 43 is controlled by the output of the calibration signal amplifier 42 to correct the electrocardiograph potential signal measured at an electrode by accounting for the electrode's state of contact.

Specifically, the signal from an electrode 44 is amplified by the first stage buffer amplifier 17, passes through the switch 16, and is input to either the electrocardiograph amplifier 43 or the calibration signal amplifier 42. After the detected electrocardiograph potential is corrected by controlling the gain of the electrocardiograph amplifier 43 with the signal output from the calibration signal amplifier 42, it is compared with the body's reference voltage in the output stage differential amplifier 18.

However, since the calibration signal and the electrocardiograph potential are time division multiplexed in this circuit, a sample and hold device 47 is connected to the output side of the diode 46. The sample and hold device 47 provides temporary memory for the output of the calibration signal amplifier 42, and uses this stored voltage to control the gain of the electrocardiograph amplifier 43. When the input select switch is moved to the calibration signal amplifier position by the control circuit 14, the sample and hold device 47 is instantly reset (memory is set to zero), and the voltage level input from the diode 46 is subsequently stored.

The output stage differential amplifier 18 amplifies the difference between the sum of the potential detected at both arms and one leg, as in the system of FIG. 1, and the output from the electrocardiograph amplifier 43. However, although it is not illustrated, the electrocardiograph amplifier 43 may be a differential amplifier which amplifies the electrocardiograph potential with respect to the reference voltage.

Incidentally, the time interval during which the calibration signal is applied to the human body by the calibration signal generator 41 and amplified by the calibration signal amplifier 42, is desirably set longer than one calibration signal period. This is for the purpose of eliminating any direct current components from the electrocardiograph potential induced at the electrodes 44. When a metal electrode 44 contacts the surface of the human body, sweat and other conducting body fluids form local battery cells which produce direct current (DC) components that are included in the electrocardiograph potential signal. The voltage produced by these local battery cells changes greatly depending on the state of contact between the electrodes 44 and the body surface. Consequently, the DC component included in the electrocardiograph potential induced at an electrode 44 varies, causing the zero level of the electrocardiograph potential to also vary. The effects of the DC component of the electrocardiograph potential picked up at an electrode 44, can be eliminated through the use of alternating current as the calibration signal, and by measuring the difference between the positive and negative voltage peaks, or amplitude of that calibration signal. In other words, even though the zero level may vary, the calibration signal peak to peak voltage will not change.

Even when a half cycle sine wave starting and finishing at zero volts is used at the calibration signal, DC component effects can be eliminated. Namely, by forcing the calibration signal voltage measured at an electrode to be zero at the start and finish of one half cycle, the effect of DC components in the signal measured at that electrode can be eliminated. For example, as shown in FIG. 6, when the voltage at the start of the calibration signal half cycle measured at an electrode 44 is 100 mV, and is 150 mV at the end of one half cycle, the DC component variation can be eliminated by forcing the starting 100 mV and the half cycle 150 mV to zero volts.

Further, even during calibration signal measurement, electrocardiograph potential is present at the body surface, and therefore, electrocardiograph potential is added into the calibration signal induced in an electrode. However, when the frequency of the calibration signal is made sufficiently high with respect to the electrocardiograph signal, and the peak to peak voltage amplitude of the calibration signal is detected at the electrode, measurement error due to variation in the zero level of the calibration signal caused by the electrocardiograph potential can be eliminated.

Since the calibration signal and the electrocardiograph signal cannot be simultaneously measured by the sytem shown in FIG. 4, restrictions are imposed on the time interval for electrocardiograph signal detection by the time required for calibration signal detection. Specifically, the electrocardiograph signal cannot be measured when the calibration signal is being detected and the state of contact of an electrode is being determined. Consequently, it is best to make the calibration signal detection time interval short, and the electrocardiograph signal measurement interval long. The calibration signal detection interval can be made short by increasing the frequency of the calibration signal generator 41. Accordingly, the frequency of the calibration signal generator 41 is made as high as possible, while considering amplifier high frequency limitations, in order to reduce the length of the calibration signal detection time interval.

However, depending on the measurement method, for detection of an electrocardiograph waveform which repeats at regular intervals, the calibration signal detection interval can be made long without imposing restrictions on electrocardiograph waveform detection. Namely, after the calibration signal is detected and the state of contact of the electrode is determined, one to several beats of the electrocardiograph signal can be measured.

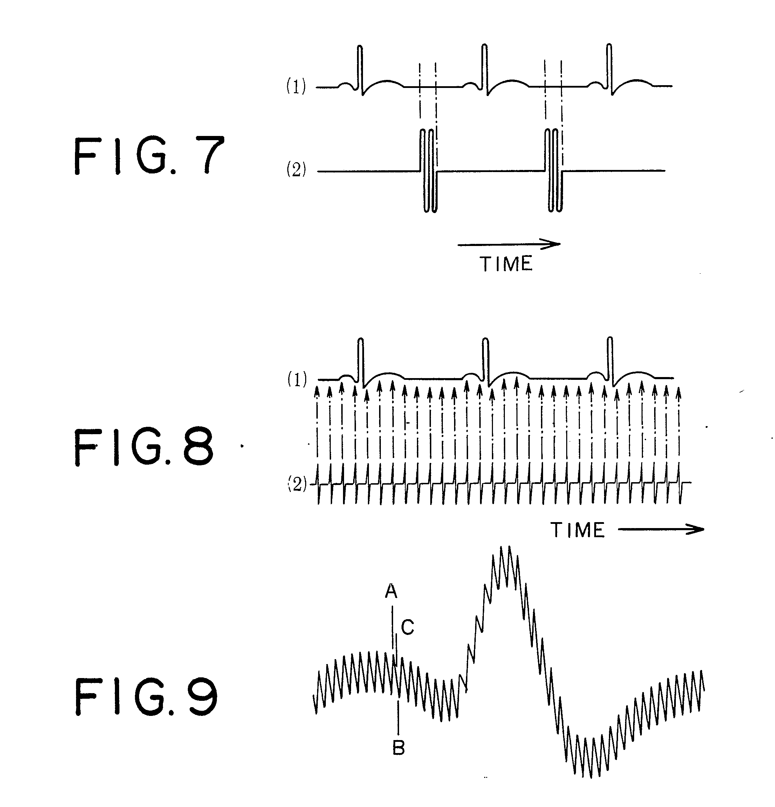

Turning now to FIG. 7 and FIG. 8, the time frame of the application of the calibration signal in relation to the electrocardiograph signal is illustrated. In these diagrams, (1) shows the electrocardiograph waveform induced in an electrode, and (2) shows the calibration signal. The horizontal axis represents time, and the vertical axis represents voltage. As shown in the method of Fig. 7, the electrocardiograph waveform (1) is at approximately the zero level during the interval that the calibration signal (2) is applied to the body. With this method, the calibration signal essentially does not restrict electrocardiograph signal measurement time. That is to say, that during the time interval when there is almost no electrocardiograph potential, conventional electrocardiograph instrumentation does not perform measurements. In this method, the calibration signal is applied to the body, the state of contact of the electrode is determined, then the electrocardiograph signal is measured for a certain interval (for example, one heart beat), and the measured value of the electrocardiograph signal is corrected by the previously determined calibration signal level. This method is appropriate for measuring elecrocardiograph potential continuously for a prescribed time period, or for measurement during extremely short time intervals (for example, from several μsec to several msec).

It turns out that in electrocardiograph measurement, the state of contact of an electrode often does not change radically. Specifically, when the electrode to body contact is poor, the entire electrocardiograph waveform is often reduced by an approximately constant percentage over a certain time interval. Accordingly, as shown in FIG. 7, when measuring electrocardiograph potential by applying the calibration signal to the body with the electrocardiograph signal essentially at its zero level, determining the state of contact of the electrode, measuring the electrocardiograph voltage near the peak of the next electrocardiograph signal, and correcting the overall electrocardiograph signal with the previously determined calibration signal and the peak voltage, normally large errors are not introduced.

Reduction of the time between measurement of the electrode's state of contact and detection of the electrocardiograph potential is an effective way to increase the accuracy of electrocardiograph measurement. This can be realized by measuring the electrode's state of contact immediately prior to each electrocardiograph potential measurement.

In the measurement method shown in FIG. 8, the calibration signal shown in (2) is applied to the human body for extremely short intervals. In this method the calibration signal is applied to the body, the electrode's state of contact is measured, and then the application of the calibration signal is ceased while the electrocardiograph potential is measured. The electrocardiograph potential can thus be detected and corrected immediately after the electrode's state of contact has been measured. Accordingly, even if the electrode's state of contact varies over the interval of one heartbeat, the electrocardiograph potential can be accurately corrected.

In this method of measurement, the time interval in which the calibration signal is applied to the body is shorter than the sampling time required for measurement of the electrocardiograph potential. For example, suppose the electrocardiograph potential is measured every millisecond, and the time required for measurement of the electrocardiograph potential (the time required for such processing as analog to digital conversion or sample and hold memory storage time) is 100 $\mu$sec. For this case, the interval during which the calibration signal is applied to the body is chosen to be less than 900 $\mu$sec.

A sine wave with a period of 900 $\mu$sec has a frequency of approximately 1.1 kHz. Therefore, allowing some margin, it is desirable to use a calibration signal with a frequency greater than 1.5 kHz. A calibration signal of this frequency can be sufficiently amplified with an inexpensive operational amplifier. If a 15 kHz calibration signal is used, detection of more than ten cycles of the calibration signal can be performed in the 900 $\mu$sec interval.

In the configuration shown in FIG. 4, where the calibration signal flows through the body, it appears that the calibration signal induced in an electrode would depend on the electrode's position on the body. However, in actual experiments performed by the inventor and others; when the calibration signal generator 41 was connected between one leg and both arms as shown in FIG. 4, and the signal was detected by electrodes on the chest, there was surprisingly no difference in calibration signal amplitude from one electrode to the next. It is conjectured that the human body acts as a single conducting solid with respect to calibration signal flow. The contact resistance R between the calibration signal generator 41 and the body surface is then much greater than the resistance of the body as a whole, so the entire body attains the same calibration signal voltage. For this reason, when all electrodes are in proper contact with the body surface, all electrodes detect the calibration signal with equal amplitude. Consequently, the state of electrical contact of all electrodes can be measured without adjusting the calibration signal detected at each electrode for that electrode's position.

It is also possible to simultaneously measure the calibration signal and the electrocardiograph potential even when the state of electrode contact is measured by calibration signal flow through the body, as shown in FIG. 4. This is realized by continuously flowing calibration signal current through the body. The electrocardiograph signal with the calibration signal added is detected at an electrode. As shown in FIG. 9, the signal detected at the electrode is the electrocardiograph waveform varying with the superposed calibration signal. The calibration signal is extracted from this signal by passage through a band pass filter which only passes the calibration signal frequency. The signal output from the band pass filter is proportional to the amplitude of the signal detected at the electrode. Consequently, the state of contact of the electrode can be measured by the output signal from the band pass filter. The electrocardiograph signal can be detected, as shown in FIG. 9, by extracting the midpoint C between the positive and negative peaks A and B. To measure the midpoint voltage C, synchronized with the calibration signal, adjacent positive and negative peak voltages are detected, and then their average value is found, or the midpoint of the local positive and negative peaks is detected.

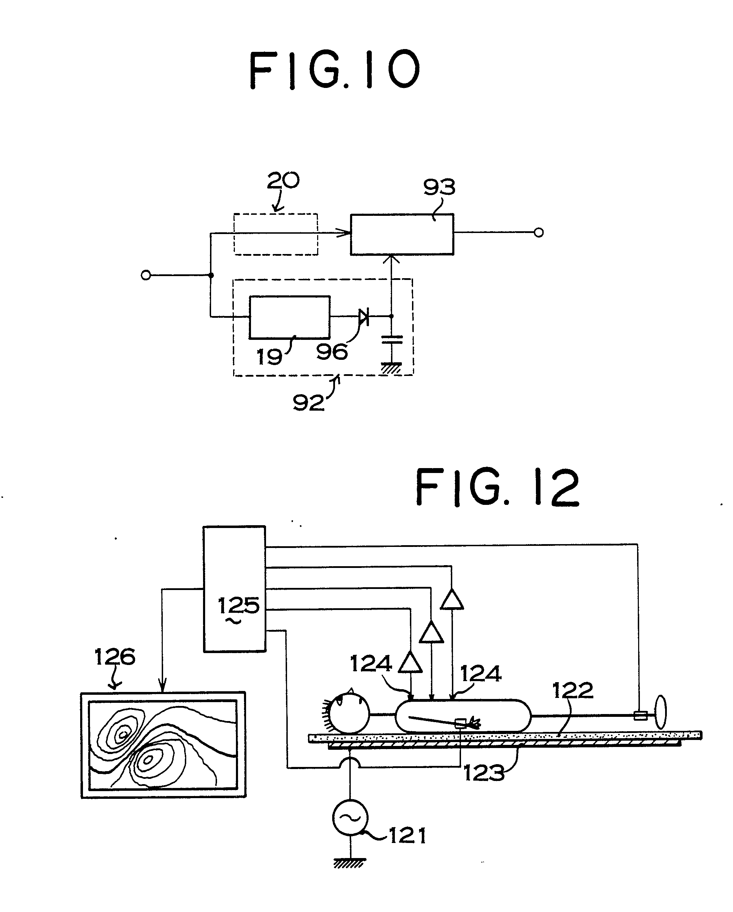

In place of FIG. 5, a circuit which can simultaneously detect the calibration signal and the electrocardiograph signal is shown in FIG. 10. This circuit is provided with a calibration signal amplifier 92 having a band pass filter 19, and an electrocardiograph amplifier 93. This circuit can be used in place of the amplifier shown in FIG. 5. However, when this circuit is used, the calibration signal is continuously applied to the body, so there is no particular need for the switching means 15 between the calibration signal generator and the body, or for the input selector switch 16 and the control circuit 14.

In the circuit of FIG. 10, the calibration signal is detected from the signal picked up at an electrode by passage through the band pass filter 19. The output from the band pass filter 19 is rectified by the diode 96 to obtain an output signal proportional to the amplitude of the calibration signal. At the same time, the signal picked up at the electrode is amplified by the electrocardiograph amplifier 93. Although it is not illustrated, a synchronization signal from the calibration signal generator is also input to the electrocardiograph amplifier 93. Using the synchronization signal from the calibration signal generator, the electrocardiograph amplifier 93 outputs the voltage of the positive and negative peaks of the superposed calibration signal, or the value of the signal at the midpoint of local positive and negative peaks. When positive and negative peak values are output, the mean value of that signal can be found by analog to digital conversion.

As shown by the broken lines of FIG. 10, it is also possible to detect the electrocardiograph signal by eliminating the calibration signal through a band elimination filter 20 connected to the input side of the electrocardiograph amplifier 93.

In the same manner as previously discussed methods, the electrocardiograph signal picked up at an electrode is corrected by the calibration signal amplifier, which is an automatic gain control circuit for the electrocardiograph amplifier 93.

The circuits shown in FIG. 1 and FIG. 4 correct and amplify the electrocardiograph signal based on the calibration signal detected at an electrode. However, this invention is not limited to correcting the electrocardiograph signal with the calibration signal detected at an electrode. In the simplest case, the calibration signal measured at an electrode can be amplified and displayed directly on a video monitor. The state of contact of the electrode can be investigated by viewing the monitor, and after good signal detection conditions have been confirmed at all electrodes, the electrocardiograph signal can be measured.

Incidentally, this invention is most effectively applied to body surface electrocardiograph systems, which measure body potential at a multiplicity of points, computer process the measured data, and display computed results on a video monitor or printer. A body surface electrocardiograph system measures the electrocardiograph signals produced in the chest region, and calculates, for display, an equipotential map of the body surface. A body surface electrocardiograph system typically measures electrocardiograph signals simultaneously from 50 to several hundred electrodes.

A body surface electrocardiograph system stores electrocardiograph signals, measured at the electrodes, in computer memory for computation. In this case, it is possible to measure the calibration signal in the body through all the electrodes prior to electrocardiograph signal measurement, store the measured calibration signals in memory, and use these memory values to correct the electrocardiograph signals measured immediately after the calibration signal measurement. For example, a calibration signal of 1 Hz or greater can be applied to the body, measured at all electrodes, amplified with a fixed gain, converted from analog to digital form, and loaded into memory. Calibration signal application to body can be immediately ceased, electrocardiograph signals can then be measured at all electrodes, amplified with a fixed gain, converted from analog to digital form, and loaded into memory. The electrocardiograph signal values in memory can then be corrected using the calibration signal values in memory (by software). Specifically, an electrocardiograph signal measured at an electrode where the calibration signal level is half the prescribed value, is multiplied by a factor of two, and an electrocardiograph signal measured at an electrode where the calibration signal value is one third that prescribed, is multiplied by a factor of three.

Although the circuits shown in FIG. 1 and FIG. 4 utilize automatic gain control circuits to correct the electrocardiograph signals, it is also possible to correct the electrocardiograph signals using computer computation. In this case, the calibration signals and the electrocardiograph signals are both stored in computer memory, and the calibration signal values are used by computer algorithms to correct the electrocardiograph values.

The circuit shown in FIG. 1 is capable of applying a calibration signal without causing appreciable current to flow through the body. In this arrangement, the calibration signal generator 1 is connected between ground and the body, and the calibration signal is applied such that the entire body attains the same voltage levels with respect to ground. The state of contact of an electrode can be measured by detection of the calibration signal at an electrode as a signal with respect to ground.

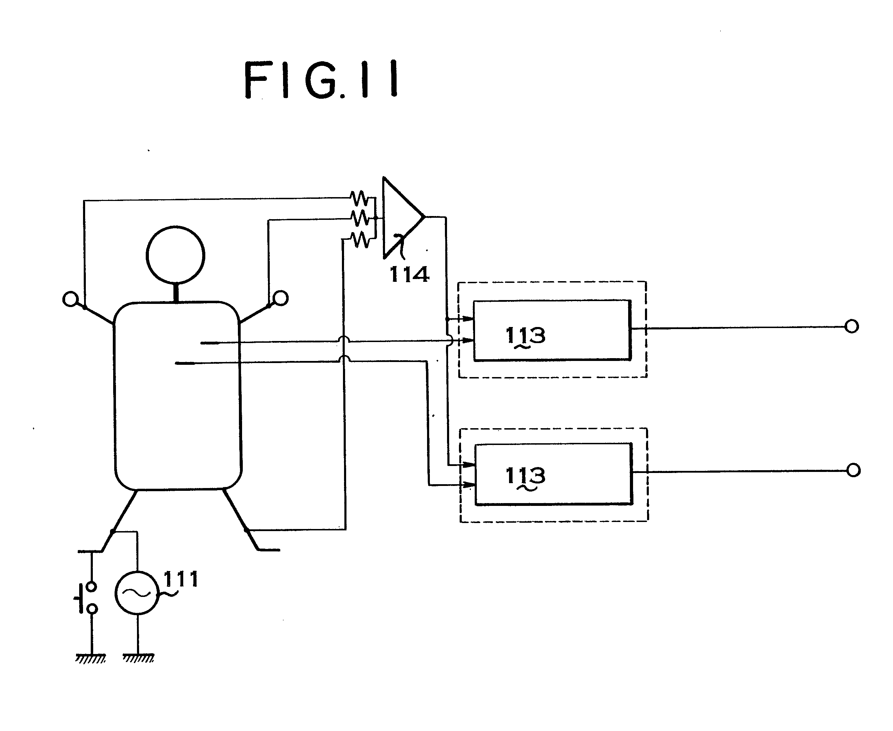

As shown in FIG. 11, it is also possible to connect the calibration signal generator 111 between ground and one leg, and amplify the calibration signal with the electrocardiograph amplifier 113. Since the entire body attains uniform potential levels which fluctuate with the calibration signal in this circuit, in other words since the body as a whole reaches the same voltage with respect to the calibration signal, the inventor and others expected that the electrocardiograph amplifier 113 would not be able to measure the calibration signal. Namely, since the electrocardiograph amplifier 113 measures the relative potential between the chest region, and both arms and one leg, if the entire body's potential fluctuates in unison, the electrocardiograph amplifier 113 should not be able to measure the calibration signal.

However, surprisingly when the inventor and others performed actual experiments, the electrocardiograph amplifier 113 proved capable of measuring the calibration signal. The reason why the electrocardiograph amplifier 113 can measure the calibration signal, is because the unity gain adder 114 which measures the potential of both arms and one leg is not an ideal operational amplifier with infinite input impedance. Accordingly, if current flows into the adder 114, the signal actually input to the adder 114 is somewhat less than the calibration signal applied to the leg. When current is input to the adder 114, a voltage drop is produced which is equal to the input current times the resistance between the adder 114 input terminal and the body, and consequently a calibration signal voltage reduced by this voltage drop is input to the adder 114. In other words, the signal actually input to the adder 114 is not at the same potential as the body. The difference between the calibration signal applied to the body and the signal actually input to the adder 114 is equal to the input current times the input side resistor. The input side resistor value is fixed, but since the adder 114 input current is proportional to the calibration signal, the difference between the actual input signal to the adder 114 and the calibration signal, is proportional to the calibration signal. The electrocardiograph amplifier 113 compares the adder 114 output signal with an electrode voltage and amplifies the difference. If the actual input voltage of the adder 114 is different than the body potential, one input to each electrocardiograph amplifier 113 will have a voltage different from that of the calibration signal. The electrocardiograph amplifier 113 can thereby measure the calibration signal.

This circuit has the feature that the calibration signal can be measured simply without switching connections to the electrocardiograph amplifier 113.

Distorted waveforms may also be used as the calibration signal applied to the body. By the simplest method, a calibration signal can be applied without the use of a signal generator or oscillator. Several centimeters to several meters of wire can be used to generate a calibration signal. The wire can be connected to the body at a position of calibration signal application. The wire connected to the body acts as an antenna and induces noise from the power supply. This induced noise has the same frequency as the power supply , and is a distorted sign wave. Although noise induced from the power supply is distorted, it can be used as a calibration signal because it can cause the potential of the entire human body to fluctuate as a whole. This is the simplest method of applying a calibration signal to the human body.

As shown in FIG. 12, the calibration signal can also be applied to the body without directly connecting the calibration signal generator 121 to the body. Specifically, as illustrated in FIG. 12, a field effect electrode 123 is provided under the body, and acts on the body through an insulating material 122, such as a synthetic resin. The calibration signal is applied between the field effect electrode 123 and ground, and the calibration signal is induced in the body from the field effect electrode 123. The calibration signal induced in the body can be measured by an electrode 124, and the state of electrical contact of the electrode 124 can be determined. In this case the calibration signal is induced in the body through the capacitor formed by the field effect electrode 123, the insulating material 122, and the body.

The system shown in FIG. 12 is provided with a computation means 125 for operating on the electrocardiograph signal detected at an electrode 124, and a video monitor 126 for displaying the computed results. The computation means 125 operates on the electrocardiograph signal, and produces a potential distribution map of the body surface. The computation means 125 acquires electrocardiograph signal data simultaneously from all electrodes at a fixed time interval, for example, between 0.1 msec to 5 msec. The acquired electrocardiograph signal data is processed at fixed intervals to compute successive potential distribution maps of the body surface. The video monitor 126 successively displays the computed potential distribution maps at, for example, 0.1 sec to 10 sec intervals.

Although for clarity, FIG. 12 shows three electrodes in contact with the body surface, as previously stated, electrocardiograph systems which produce body surface potential distribution maps are provided with a multiplicity of electrodes.

The electrocardiograph system shown in FIG. 1 is provided with a video monitor 25, which is part of the computation means. This video monitor displays body surface potential distribution maps just as the computation means and video monitor of FIG. 12 do.

Incidentally, the method for potential measurement on a living body of this invention is not limited to a body potential measurement means which must be in contact with the body. Any material or device which can detect an electrical signal from the body, for example, a non contacting electrode which measures body potential by being in close proximity to, but not in electrical contact with the body, can also be used. Non contacting electrodes attach to the body surface, and electrically connect with the body through some capacitance. In other words, a non contacting electrode measures body potential through a capacitor which has the electrode and the body surface as plates. In this case, the electrode cannot measure direct current levels from the body, but alternating current components pass through the electrode's capacitor and are detected. In an electrode of this configuration, as the area of the body surface covered by the electrode is increased, while increasing the input impedance of the first stage amplifier connected to the electrode, the capacitance increases allowing lower frequency signals to be detected. Namely, the contact impedance of this type of electrode is inversely proportional to the body signal frequency and, for a given dielectric, the electrode surface area.

Further, a magnetic sensor, such as a SQUID (superconducting quantum interference device), can be used as a body signal detection device for detecting magnetic fields produced by the body. The magnetic sensor is placed in close proximity to the body surface to measure the body's magnetic field. In this case, a magnetic signal which can be detected by a magnetic sensor is used as the calibration signal applied to the body.

## PRACTICALLY OF THE INVENTION WITHIN THE INDUSTRY

The methoed for potential measurement on a living body of this invention is particularly suited for measuring induced potential at a multiplicity of points on the human body. Simultaneously maintaining all of the electrodes at a multiplicity of points on the human body in a state of contact favorable to body potential measurement is difficult. It is also difficult to determine the state of contact of an electrode just from the body potential signal picked up at that electrode. In this invention, a calibration signal is applied to the human body. The applied calibration signal is detected at each electrode, and the calibration signal picked up by an electrode indicates the electrical state of contact of that electrode. Hence, the state of electrical contact to the human body of a multiplicity of electrodes can be investigated by detection of a calibration signal applied to the body. The method of this invention is, therefore, effective with the use of numerous electrodes for the measurement of signals from a living body.

## Claims

1. A method for potential measurement on a living body, wherein an electrode is put in contact with, or very close to the body, and electrical signals originating in the body are detected by the electrical potential induced in said electrode, characterized in that body signal detection conditions at said electrode are examined by applying a calibration signal to the body, and detecting said calibration signal at said electrode.

2. A method for potential measurement on a living body, wherein an electrode is put in contact with, or very close to the body, and electrical signals originating in the body are detected by the electrical potential induced in said electrode, characterized in that measurement values of the body potential are corrected by applying a calibration signal to the body, and detecting said calibration signal at said electrode.

3. A method for potential measurement on a living body as defined in claim 1 and claim 2, wherein alternating current is used as the calibration signal.

4. A method for potential measurement on a living body as defined in claim 3, wherein alternating current with a frequency equal to that of the supply power is used as the calibration signal.

5. A method for potential measurement on a living body as defined in claim 1 and claim 2, wherein the calibration signal is applied to the body with respect to ground.

6. A method for potential measurement on a living body as defined in claim 1 and claim 2, wherein the calibration signal is applied at at least two points on the body.

7. A method for potential measurement on a living body as defined in claim 1 and claim 2, wherein the calibration signal applied to the body is detected at forty or more electrodes.

# FIG. 1

# FIG. 2

EP 0 335 977 A1

POTENTIAL ———— (1)

POTENTIAL ———— (2)

POTENTIAL ———— (3)

POTENTIAL ———— (4)

TIME →

# F I G. 3(A)

POTENTIAL ———— (1)

POTENTIAL ———— (2)

POTENTIAL ———— (3)

POTENTIAL ———— (4)

TIME →

# F I G. 3(B)

FIG. 4

FIG.6

FIG.5

FIG. 7

TIME

FIG. 8

TIME

FIG. 9

# FIG. 10

# FIG. 12

# FIG.11

114

113

113

111

EP 0 335 977 A1

# INTERNATIONAL SEARCH REPORT

International Application No  PCT/JP88/00906

## I. CLASSIFICATION OF SUBJECT MATTER (if several classification symbols apply, indicate all) [6]

According to International Patent Classification (IPC) or to both National Classification and IPC

Int.Cl[4]    A61B5/04

## II. FIELDS SEARCHED

| Minimum Documentation Searched [7] | |
| --- | --- |
| Classification System | Classification Symbols |
| IPC | A61B5/04 |

| Documentation Searched other than Minimum Documentation to the Extent that such Documents are Included in the Fields Searched [8] |
| --- |
| Jitsuyo Shinan Koho              1926 - 1988 |
| Kokai Jitsuyo Shinan Koho        1971 - 1988 |

## III. DOCUMENTS CONSIDERED TO BE RELEVANT [9]

| Category [*] | Citation of Document, [11] with indication, where appropriate, of the relevant passages [12] | Relevant to Claim No. [13] |
| --- | --- | --- |
| X | JP, Y2, 55-12803 (Dia Medical System Kabushiki Kaisha) 22 March 1980 (22. 03. 80) Pages 1 to 3 (Family: none) | 1, 3-7 |
| X | JP, B2, 52-17352 (Matsushita Electric Ind. Co., Ltd.) 14 May 1977 (14. 05. 77) Pages 1 to 4 (Family: none) | 2, 3-7 |
| A | JP, A, 53-83378 (Electronics For Medicine Inc.) 22 July 1978 (22. 07. 78) Pages 1 to 6 & US, A, 4112930 & GB, A, 1589338 | 7 |

* Special categories of cited documents: [10]

"A"  document defining the general state of the art which is not considered to be of particular relevance

"E"  earlier document but published on or after the international filing date

"L"  document which may throw doubts on priority claim(s) or which is cited to establish the publication date of another citation or other special reason (as specified)

"O"  document referring to an oral disclosure, use, exhibition or other means

"P"  document published prior to the international filing date but later than the priority date claimed

"T"  later document published after the international filing date or priority date and not in conflict with the application but cited to understand the principle or theory underlying the invention

"X"  document of particular relevance; the claimed invention cannot be considered novel or cannot be considered to involve an inventive step

"Y"  document of particular relevance; the claimed invention cannot be considered to involve an inventive step when the document is combined with one or more other such documents, such combination being obvious to a person skilled in the art

"&"  document member of the same patent family

## IV. CERTIFICATION

| Date of the Actual Completion of the International Search | Date of Mailing of this International Search Report |
| --- | --- |
| November 22, 1988 (22. 11. 88) | December 5, 1988 (05. 12. 88) |
| International Searching Authority | Signature of Authorized Officer |
| Japanese Patent Office | |

Form PCT/ISA/210 (second sheet) (January 1985)